Europäisches Patentamt

European Patent Office

Office européen des brevets

(19)

(11) Publication number: **0 060 385**
**B1**

(12) **EUROPEAN PATENT SPECIFICATION**

(45) Date of publication of patent specification: **15.08.84**

(51) Int. Cl.³: **A 61 B 5/14**

(21) Application number: **82100819.0**

(22) Date of filing: **05.02.82**

(54) Needle assembly with vein entry indicator.

(30) Priority: **16.03.81 US 244409**

(43) Date of publication of application:
**22.09.82 Bulletin 82/38**

(45) Publication of the grant of the patent:
**15.08.84 Bulletin 84/33**

(84) Designated Contracting States:
**BE DE FR GB IT NL SE**

(56) References cited:
**EP - A - 0 042 211**
**EP - A - 0 049 591**
**DE - C - 411 474**
**FR - A - 2 379 293**
**GB - A - 2 020 425**
**US - A - 4 312 362**

(73) Proprietor: **Becton, Dickinson and Company**
**Mack Centre Drive**
**Paramus New Jersey 07652 (US)**

(72) Inventor: **Kaufman, Joseph**
**10 Crest Road**
**Emerson New Jersey (US)**

(74) Representative: **Selting, Günther, Dipl.-Ing. et al,**
**Deichmannhaus am Hauptbahnhof**
**D-5000 Köln 1 (DE)**

Courier Press, Leamington Spa, England.

## Description

### 1. Field of the invention

The present invention relates to a needle assembly for collecting fluid, and more particularly, concerns a needle assembly for collecting a single sample of blood from a patient while providing an indication of entry of the needle assembly into the vein of the patient.

### 2. Description of the prior art

In the collection of body fluids, such as blood from a patient, the nurse or clinician collecting this fluid oftentimes must probe for or guess the location of the patient's vein. For instance, the nurse attempting to collect a blood sample, even with tourniquet pressure applied, cannot readily insert the needle in patients who have small veins or if the veins are not close to the skin surface. In those instances, the needle may either miss the vein entirely or perhaps may pass completely through a thin vein. As a result, blood flow through the needle into the evacuated blood collection tube would be uncomfortable and inefficiently slow since the blood would not be flowing from the patient's vein. The nurse would have to start over again and perhaps use another evacuated blood collection tube on the next attempt which, of course, would be wasteful of both materials and expense, not to mention the discomfiture which the patient experiences.

In order to reduce the waste of evacuated blood collection tubes which occurs in the blood collecting procedure where the nurse must guess at vein location, a number of blood collecting devices have been proposed which provide an indicator to the user that satisfactory vein entry has been accomplished. In these visual indicator devices, an antechamber is generally provided which is kept isolated by some sort of valving mechanism from the opening of the needle which deposits the blood being collected into the evacuated tube. Once the nurse probes for the vein and satisfactory entry is made, blood is supposed to flow through the needle into the antechamber to provide a telltale trace that the vein has been properly located. At this point in the blood collecting procedure, an evacuated blood collection tube is then usually inserted into the blood collection holder, opening up some type of valve whereby blood is then free to flow into the evacuated blood collection tube. Typical of these visual indicator blood collection devices are those found in U.S. Patent Nos. 4,166,450 and 3,585,984.

Although, in theory, this antechamber approach for the telltale trace of blood flow is workable, and oftentimes may work satisfactorily, there are, nevertheless, some inherent problems with this approach. For example, the antechamber generally is filled with air at the time the forward end of the needle is inserted into the vein of the patient.

Inasmuch as the opposite end of the needle, for puncturing the evacuated blood collection tube, is valved or closed, the air in the antechamber forms a blockage since there is no place for such air to be displaced. Thus, even if the nurse locates the vein, and satisfactory entry is made with normal tourniquet pressure, the blood flow from the patient may not travel all the way through the needle before reaching the antechamber. This is due to the fact that the air in the antechamber blocks the blood flow through the needle even under normal tourniquet pressure. The net result of this is that the nurse will be deceived into believing that satisfactory vein entry has not been accomplished since no telltale trace will be visually observed. Unnecessary secondary venipuncture may take place which, again, is not only uncomfortable to the patient but is inefficient and wasteful.

Accordingly, improved approaches for providing a visual indicator to the user of a blood collecting device are still being sought. These improvements are needed in those types of blood collecting devices which make entry into the vein of the patient before the evacuated blood collection tube is inserted into the blood collection holder, which now is a very common blood collecting procedure.

The earlier EP—A—0049591 that shall be considered as comprised in the state of the art according to Art. 54(3) EPC discloses a needle assembly comprising a housing with a chamber therein and having first and second access openings therethrough in fluid communication with the chamber, the first access opening being in fluid communication with a first cannula and the second access opening being in fluid communication with a vacuum collection device by means of a second cannula extending inwardly into the chamber with a closed end and having a pair of holes longitudinally spaced from each other and from said second access opening and comprising a slidable plug for slidably plugging the chamber to prevent liquid from flowing into one of said holes while allowing gas to flow into one of said holes at a first position of the plug in the chamber whereby the prevented liquid may be viewed by a user through viewing means of the housing, the slidable plug being responsive to a negative pressure gradient at said second access opening to slide to a second position in the chamber to allow liquid to flow into said one of said holes whereby liquid may be collected from said second access opening.

### Summary of the invention

A needle assembly for determining fluid access when collecting a fluid sample from a source of fluid comprises a housing with a chamber therein and having first and second access means therethrough in fluid communication with the chamber. The housing includes means for viewing the contents inside the chamber.

Means extends into the chamber for directing passage of gas and liquid into the second access means. Means operatively plugs the extension means to delay liquid from flowing into the extension means while allowing gas to flow into the extension means. The delayed liquid may be viewed by a user through the viewing means. Included with the plug means is liquid soluble sealant means removable from the extension means upon contact with the liquid. This allows liquid to flow into the extension means whereby it may be collected from the second access means.

In a preferred embodiment of the needle assembly of the present invention as generally set forth above, the housing is translucent and has a forward end, a rearward end and a chamber within. A first cannula extends outwardly from the forward end of the housing in fluid communication with the chamber. A second cannula has an exterior portion extending outwardly from the rearward end of the housing and also has an interior portion extending inwardly into the chamber with an open distal end and a side hole therethrough. A vent plug of air-permeable, blood-impermeable, porous material is in liquid-tight contact with the outer surface of the interior poriton of the second cannula. This vent plug overlies the side hole and preferably forms a pocket around the open end of the interior portion of the second cannula. A blood soluble mass of sealant plug material is positioned in the pocket and covers the open distal end of the interior portion. Under normal tourniquet pressure, blood flows into the chamber from the first cannula and is delayed by the sealant plug from flowing into the second cannula but can be viewed by a user through the translucent housing to indicate vein entry by the first cannula. Any air initially in the chamber passes through the vent plug and into the side hole for removal from the assembly. The sealant plug is adapted to be solubilized into the blood inside the chamber to thereby uncover the open end of this second cannula. As a result, blood may flow through the open distal end and into the second cannula for collection into the blood collection container.

In accordance with the principles of the present invention, there are structural elements and features herein which are notably different from the prior inventions of this type. For instance, the vent and sealant plugs inside the chamber and against the interior cannula serve as a valve mechanism to prevent blood from flowing out of the chamber when the patient needle has been inserted into the vein of the patient. At this point of the blood collecting procedure, the evacuated blood collection tube is normally not yet in position. Therefore, at this point blood should not be allowed to escape from the needle assembly. However, once the nurse makes entry into the patent's vein blood will flow through the forward needle and enter the chamber inside the needle assembly. The nature of the plug materials will prevent any blood flow through the chamber. However, inasmuch as the vent plug material is air-permeable, air either initially in the chamber or in the forward needle will be allowed to escape from the chamber and needle assembly to eliminate the blockage which occurs in prior art needle assemblies proposed for visual indicator operation. Moreover, the sealant plug material is removable from the interior cannula inside the chamber due to the blood soluble character of this sealant plug. Blood inside the chamber solubilizes the sealant plug thereby exposing the open distal end in the collection cannula to the blood flow path. In this fashion, as soon as the sealant plug is solubilized, its removal from the open end effects a valve opening thereby allowing the blood to flow through the needle assembly and into the blood collection container.

Advantageously, the present invention eliminates the deficiencies of the air blockage problem as set forth above with respect to prior art needle assemblies. In this regard, the present invention allows the user to visually observe the trace of blood entering into the chamber inside the needle assembly as soon as the vein has been properly entered, since any air inside the needle assembly is allowed to escape, thereby permitting the free flow of blood into the chamber. Inasmuch as the sealant plug takes some time to solubilize, the blood is delayed from flowing out of the chamber long enough to allow the blood collection container to be attached to the assembly.

Brief description of the drawings

Fig. 1 is an exploded perspective view illustrating the preferred needle assembly, a holder for an evacuated container and an evacuated blood collection container for use in obtaining a blood sample from a patient;

Fig. 2 is an enlarged cross-sectional view taken along line 2—2 of Fig. 1;

Fig. 3 is an end view of the preferred vent plug and sealant plug for use in the chamber of the embodiment of Fig. 1 taken along line 3—3 of Fig. 2;

Fig. 4 is a perspective view of the needle assembly connected to a holder and inserted into a patient so that a user can view same for indication of vein entry; and

Fig. 5 is a cross-sectional view taken along line 5—5 of Fig. 1 with the components in an assembled condition as they would appear during use with the sealant plug solubilized so that blood can flow through the needle assembly into the collection container.

Description of the preferred embodiment

While this invention is satisfied by embodiments in many different forms, there is shown in the drawings and will herein be described in detail a preferred embodiment of the invention, with the understanding that the present disclosure is to be considered as exemplary of the principles of the invention and is not intended to limit the invention to the embodiment illustrated.

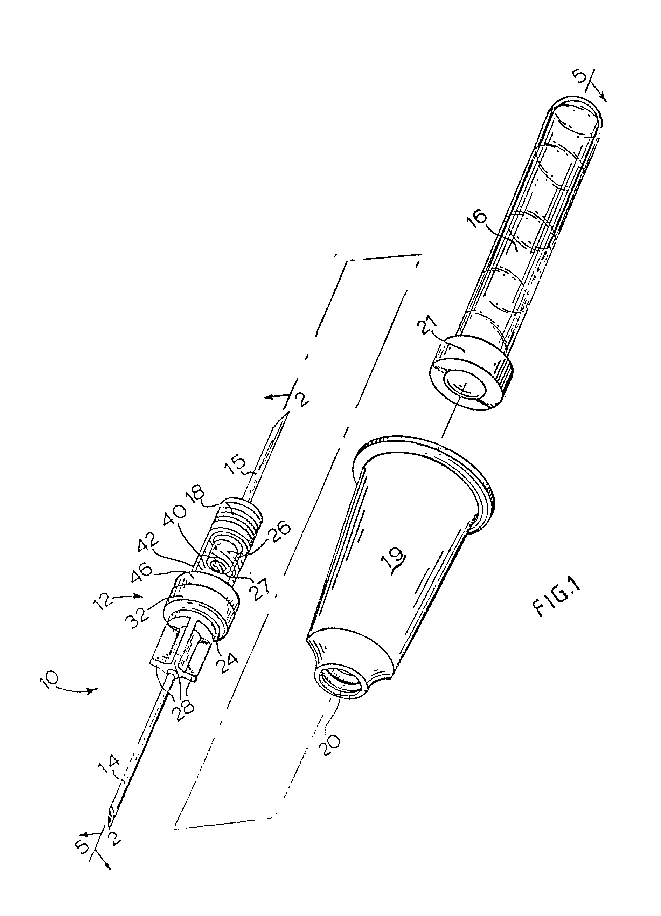

Referring to the drawings, particularly Fig. 1, there is illustrated the embodiment of a preferred single sample needle assembly 10. The basic external components of needle assembly 10 include a housing 12, a first needle cannula 14 adapted for insertion into a patient and a second needle cannula 15 at the opposite end of housing 12, the second needle cannula adapted for penetration of an evacuated container 16 for collection of a blood sample. Housing 12 includes a threaded portion 18 adjacent second cannula 15 onto which a container holder 19 is threaded by its internal mating threads 20 at the forward end of the holder. Evacuated container 16 slides into holder 19 so that second needle cannula 15 can penetrate the penetrable stopper 21 at the forward end of the evacuated container. These general aspects of blood sample collection in this type of structure are well known to those skilled in this art.

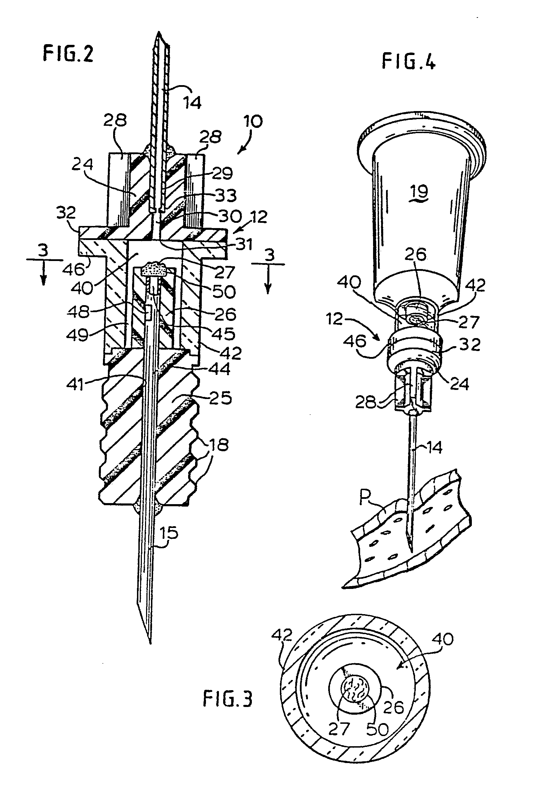

In Figs. 2 and 3, the detailed construction of needle assembly 10 is illustrated. Housing 12 has a forward end 24 and a rearward end 25, these ends being preferably separable in order to place a vent plug 26 and a sealant plug 27 in their proper positions within the housing. Forward end 24 is preferably cylindrically shaped and has a bore 29 preferably extending partially therethrough which is generally sized to slidably fit needle cannula 14 therein. In this embodiment being described, since bore 29 does not extend completely through forward end 24, a smaller diameter channel 30 communicates with bore 29 forming an access opening 31. At the junction between bore 29 and channel 30 a shoulder 33 is formed. Needle cannula 14 abuts against this shoulder for proper positioning. Once the needle cannula is in position it may be suitably affixed such as by adhesive means or the like.

Forward end 24 of the housing also includes a number of longitudinal ribs 28 surrounding the outwardly extending cannula. A needle shield (not shown) generally covers the outwardly extending needle cannula and includes mating internal ribs within. The mating ribs between needle shield and needle assembly allow the user to facilitate the insertion or removal of the needle assembly into tube holder 19. Forward end 24 also includes an annular flange 32 which serves to provide a surface for joining the two portions of the housing together upon assembly. Once again, suitable fastening means, such as adhesives or the like, may be used to secure the two portions of the housing together.

Rearward end 25 is also preferably cylindrically shaped and includes a larger bore extending partially into one end of this portion. This bore serves as a chamber 40 within housing 12. A smaller bore 41 extends through the opposite end of rearward end 25 and is substantially similar to bore 29 in the forward end of the housing. Once again, bore 41 is sized to accept the diameter of second needle cannula 15, which is secured to bore 41 by appropriate means, including adhesives and the like. The outer portion of rearward end 25 surrounding bore 41 includes external threads 18 which are provided as previously mentioned as a connection mechanism to the tube holder. The outer portion 42 of rearward end 25 surrounding chamber 40 is preferably smooth and translucent or transparent so that a user of this assembly can view the interior of the housing. In many situations, it may be preferable to make the entire rearward end, and even possibly the forward end, out of translucent or transparent material for ease of manufacture and to minimize the different types of materials which may be used in this assembly. Translucent rigid plastic is the most desirable material for inclusion in this assembly. Various sealed windows, ports or other means for a user to view the contents of the chamber are within the purview of this invention. It is preferable that such window or port be sealed so that any blood which enters chamber 40 upon the needle entering the vein will not escape from this assembly.

Second needle cannula 15 which extends outwardly from rearward end 25 preferably extends through bore 41 so that it also has an integrally formed interior portion 44 extending inwardly into chamber 40, sufficently far so that vent plug 26 is prevented from becoming disengaged therefrom. The distal end of this hollow needle cannula includes an opening 45 which is in fluid communication with the lumen of this hollow cannula. A side hole 48 is located through the side of interior portion 44 of this needle cannula and establishes fluid communication between chamber 40 and the lumen of this cannula. Rearward end 25 also includes an annular flange 46 which cooperates with flange 32 in joining the two ends of the housing together. Upon assembling forward end and rearward end together with vent plug 26 and sealant plug 27 placed in their proper positions, respective flanges 32 and 46 are secured together by appropriate fastening means, such as adhesives and the like. Vent plug 26 is placed inside chamber 40 so that it contacts the outer surface of interior portion 44 of the second cannula, in a liquid-tight engagement. Preferably, an annular gap 49 is left in chamber 40 between porous plug 26 and the wall 42 surrounding the chamber. This will allow the blood which initially enters the chamber upon venipuncture to occupy more volume thereby providing a better visual indicator to the user.

Vent plug 26 is preferably cylindrically shaped and is generally sized to fit in snug engagement around the surface of the interior cannula. This vent plug covers side hole 48 allowing air, but not blood, to pass into the side hole. The end of vent plug 26 facing toward the chamber at the distal end of the second cannula includes a pocket 50. Pocket 50 may be a counter-bore or counter-sink formed in the plug material, and is positioned so that it lies slightly above open end 45 at the distal end of the second cannula. This vent plug is made from a material intended to be gas-permeable, liquid-impermeable, and preferably air-permeable and blood-impermeable. These permeability properties would allow air to pass therethrough while preventing blood from flowing through this vent structure. Although other materials may be used, it is preferred that vent plug 26 be made of porous material, such as sintered polyethylene having a general pore rating of about ten (10) micrometers.

Sealant plug 27 is positioned over the distal end of interior portion 44 of the second cannula so that it covers and seals open end 45. Pocket 50 in the vent plug surrounds the sealant plug and facilitates its proper positioning at this distal end of the second cannula. Sealant plug 27 is preferably a mass of blood soluble material. As blood inside the chamber contacts the soluble plug, solubilization occurs, and over a period of time, the plug material is completely solubilized in the blood. Once this happens, the distal end of the second cannula will be open so that blood will be free to flow therethrough. During the time the sealant plug is solubilizing, blood flow into the second cannula will be delayed until the open end is uncovered. Although many materials may be chosen, one preferable material is polyvinylpyrollidone (PVP). For example, an 8% solution of PVP is applied across open end 45. When dry, the PVP forms a membrane over this end. In addition, when choosing the sealant plug material, it should be kept in mind that the sealant plug material should be compatible with the blood, or other collected fluid, so that no harmful effects are imparted to the blood which is to be subsequently tested.

Turning now to Fig. 4, preferred needle assembly 10 is illustrated connected to a collection holder 19. At this stage of the blood collecting procedure, evacuated tube 16 is not yet attached while cannula 14 is initially inserted into patient P. At this stage, the sealant plug is in its position within the pocket of the vent plug at the distal end of the second cannula, covering the open end and effectively sealing same. Also at this stage, since no vacuum is being applied to needle cannula 15, the chamber and needles are substantially at atmospheric pressure conditions, so that there is air in the hollow portions of the needles and the chamber. Once vein entry has been made by needle cannula 14, blood will flow under normal tourniquet pressure through cannula 14, and will fill chamber 40. The air which was in this chamber is allowed to escape through the air-permeable material of vent plug 26. Air then enters side hole 48 and escapes through second needle cannula 15 which is open at its proximal end. As a result, blood may readily fill chamber 40 with no air blockage encountered as in prior art devices. Blood in the forward chamber is delayed from flowing into the second needle cannula due to both the liquid-tight seal formed by the blood-impermeable property of vent plug 26 and by the sealant plug 27 which covers the open distal end of the second cannula. In this regard, blood in the chamber can be viewed by a user through translucent portion 42 to have a visual indication that vein entry has been achieved by cannula 14. It should be pointed out that the force of blood flowing into the chamber under normal tourniquet pressure will not be sufficient to dislodge sealant plug 27 from its initially lying position over the open end of the second cannula.

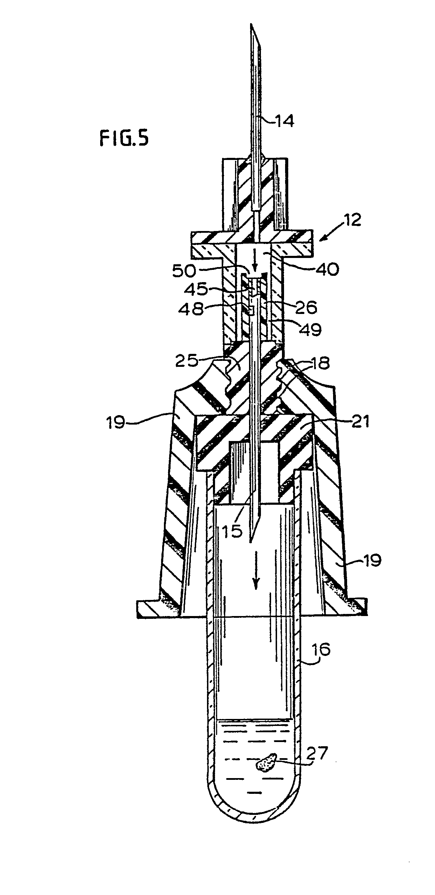

Once this visual observation of vein entry is confirmed by the user, evacuated tube 16 is then inserted into holder 19 so that penetrable stopper 21 is penetrated by hollow cannula 15. Fig. 5 illustrates this insertion of the evacuated blood collection tube. The user has time to place the collection tube into the holder since the sealant plug can be designed to solubilize relatively slowly, thereby causing a sufficiently long delay for this step to be accomplished. Once the vacuum from the collection tube is applied, additional force is applied to the blood in the chamber to draw the blood into the open end of the interior cannula. With the open end of this cannula uncovered, blood will now flow through the needle assembly and into the blood collection container until a sufficient quantity has been collected, whereby the blood sample procedure is terminated by removal of needle cannula 14 from the vein of the patient.

Thus, the needle assembly of the present invention includes vent and plug means which are easily mounted in the assembly and allow air to escape from the assembly during the initial venipuncture step as vein entry is being determined. With this air escapement feature, a blood trace upon vein entry will immediately flow into the chamber of the present needle assembly with a visual indicator feature being provided to the user to take cognizance of vein entry. With this assurance of vein entry, the blood collection container can be satisfactorily filled for collection of the sample from the patient.

**Claims**

1. A single sample needle assembly (10) for determining vein entry when collecting a blood sample from a patient comprising:

a housing (12) having a forward end (24), a rearward end (25) and a chamber (40) within, said housing (12) being translucent at least around the chamber (40) so that said chamber (40) can be viewed by a user of said assembly (10);

a first access opening (31) through the forward end (24) of said housing (12) in fluid communication with said chamber (40);

a first cannula (14) extending outwardly from said first access opening (31) in fluid communication with said chamber (40) adapted for insertion into a patient;

a second access opening (41) through the rearward end (25) of said housing in fluid communication with said chamber (40);

a second cannula (15) in fluid communication with said second access opening (41) extending inwardly into said chamber (40) with a closed end and at least two holes (45, 48) therethrough;

a vent plug (26) of air-permeable, blood-impermeable material in liquid-tight contact with the outer surface of said second cannula (15), said vent plug (26) overlying one of said holes (48); and

a blood soluble sealant plug (27) covering said second hole (45), whereby blood flowing into said chamber (40) from said first cannula (14) under normal tourniquet pressure is delayed by said sealant plug (27) from flowing into said second cannula (15) but can be viewed by a user through said translucent housing (12) to indicate vein entry by said first cannula (14) while any air initially in said chamber (40) passes through said vent plug (26) and into said first hole (48) for removal from said assembly (10), said sealant plug (27) adapted to be solubilized into the blood inside said chamber (40) to thereby uncover said second hole (45) whereby blood may flow through said second hole (45) and through said second cannula (15) for collection out of said second access opening (41).

2. The assembly of Claim 1 wherein said first hole (48) is through the side of said second cannula (15) and said second hole (45) is at the distal end of said second cannula (15).

3. The assembly of Claim 1 wherein said vent plug (26) forms a pocket (50) around the distal end of said second cannula (15) to facilitate the placement of the sealant plug (27) on said second hole (45).

4. The assembly of Claim 1 wherein said vent plug (26) material is porous.

5. The assembly of Claim 4 wherein said plug material is sintered polyethylene having a general pore rating of about ten (10) micrometers.

6. The assembly of Claim 1 wherein said vent plug (26) material is in the shape of a hollow cylinder.

7. The assembly of Claim 1 wherein said sealant plug is polyvinylpyrollidone.

8. The assembly of Claim 1 which further includes a delivery cannula extending outwardly from said second access opening (41) in fluid communication with said second cannula (15) adapted for penetration of an evacuated container (16) for collection of a blood sample.

9. The assembly of Claim 8 wherein said delivery cannula and said second cannula (15) are integrally formed in a unitary, one-piece structure.

10. The assembly of Claim 1 wherein the entire housing (12) is translucent.

11. The assembly of Claim 1 which further includes a holder (19) for an evacuated container (16) connected to said housing (12).

12. A needle assembly for determining fluid access when collecting a fluid sample from a source of fluid comprising:

a housing (12) with a chamber (40) therein and having first (31) and second (41) access means therethrough in fluid communication with said chamber (40), said housing (12) including means for viewing the contents of the chamber (40) therein;

means (44) extending into said chamber (40 for directing passage of gas and liquid into said second access means (41); and

means (26, 27) for operatively plugging said extension means (44) to delay liquid from flowing into said extension means (44) while allowing gas to flow into said extension means (44) whereby the delayed liquid may be viewed by a user through said viewing means, said plug means including a vent plug (26) of gas-permeable and liquid impermeable and preferably air-permeable and blood-impermeable material fitting in snug engagement around the surface of said extension means (44) and a liquid soluble sealant plug (27) removable from said extension means (44) upon contact with said liquid for allowing liquid to flow into said extension means (44) whereby liquid may be collected from said second access means (41).

**Patentansprüche**

1. Einzelproben-Nadelanordnung (10) zur Feststellung des Einstiches in eine Vene bei der Entnahme einer Blutprobe von einem Patienten, bestehend aus einem Gehäuse (12) mit einem vorderen Ende (24), einem hinteren Ende (25) und einer inneren Kammer (40), wobei das Gehäuse (12) wenigstens um die Kammer (40) durchsichtig ist, so daß die Kammer (40) von einem Benutzer der Anordnung (10) beobachtet werden kann;

einer ersten Zugangsöffnung (31) durch das vordere Ende (24) des Gehäuses (12), die mit der Kammer (40) in Fluidverbindung steht;
einer ersten Kanüle (14), die von der mit der Kammer (40) in Fluidverbindung stehenden ersten Zugangsöffnung (31) nach außen ragt und in einen Patienten einstechbar ist;
einer zweiten Zugangsöffnung (41) durch das hintere Ende (25) des Gehäuses, die mit der Kammer (40) in Fluidverbindung steht;

einer zweiten Kanüle (15), die mit der zweiten Zugangsöffnung (41) in Fluidverbindung steht und mit einem geschlossenen Ende und wenigstens zwei durchgehenden Löchern (45, 48) in das Innere der Kammer (40) ragt;

einem Entlüftungsstöpsel (26) aus luftdurchlässigem, blutundurchlässigem Material, der die Außenfläche der zweiten Kanüle (15) flüssigkeitsdicht berührt und der über einem der Löcher (48) liegt und

einem blutlöslichen Dichtungsstöpsel (27), der das zweite Loch (45) bedeckt, wodurch unter normalem Tourniquet-Druck aus der ersten Kanüle (14) in die Kammer (40) strömendes Blut von dem Dichtungsstöpsel (27) an einem Einströmen in die zweite Kanüle (15) gehindert wird, jedoch für einen Benutzer durch das durchsichtige Gehäuse (12) sichtbar ist, um den Veneneinstich der ersten Kanüle (14) anzuzeigen, während sämtliche zunächst in der Kammer (40) befindliche Luft durch den Entlüftungsstöpsel (26) und in das erste Loch (48) zur Entfernung aus der Anordnung (10) strömt, wobei der Dichtungsstöpsel (27) in dem Blut innerhalb der Kammer (40) löslich ist, damit das zweite Loch (45) freigelegt wird, so daß Blut durch das zweite Loch (45) und durch die zweite Kanüle (15) strömen und aus der zweiten Zugangsöffnung (41) gesammelt werden kann.

2. Anordnung nach Anspruch 1, bei der das erste Loch (48) die Seite der zweiten Kanüle (15) durchsetzt und das zweite Loch (45) sich an dem distalen Ende der zweiten Kanüle (15) befindet.

3. Anordnung nach Anspruch 1, bei der der Entlüftungsstöpsel (26) um das distale Ende der zweiten Kanüle (15) eine Tasche (50) bildet, un die Anordnung des Dichtungsstöpsels (27) auf dem zweiten Loch (45) zu erleichtern.

4. Anordnung nach Anspruch 1, bei der das Material des Entlüftungsstöpsels (26) porös ist.

5. Anordnung nach Anspruch 4, bei der das Stöpselmaterial gesintertes Polyethylen mit einer generellen Porenbemessung von etwa zehn (10) Mikrometern ist.

6. Anordnung nach Anspruch 1, bei der das Material des Entlüftungsstöpsels (26) die Form eines hohlen Zylinders aufweist.

7. Anordnung nach Anspruch 1, bei der der Dichtungsstöpsel Polyvinylpyrollidon ist.

8. Anordnung nach Anspruch 1, die außerdem eine von der zweiten Zugangsöffnung (41) nach außen ragende Abgabekanüle aufweist, die mit der zweiten Kanüle (15) in Fluidverbindung steht und zum Eindringen in einen evakuierten Behälter (16) zur Sammlung einer Blutprobe eingerichtet ist.

9. Anordnung nach Anspruch 8, bei der die Abgabekanüle und die zweite Kanüle (15) vollständig in einheitlicher einstückiger Gestaltung ausgebildet sind.

10. Anordnung nach Anspruch 1, bei der das gesamte Gehäuse (12) durchsichtig ist.

11. Anordnung nach Anspruch 1, die außerdem einen Halter (19) für einen mit dem Gehäuse (12) verbundenen evakuierten Behälter (16) aufweist.

12. Nadelanordnung zur Feststellung des Fluidzutritts bei der Sammlung einer Fluidprobe von einer Fluidquelle, bestehend aus einem Gehäuse (12), in dem sich eine Kammer (40) befindet und durch das erste (31) und zweite (41) Zugangsmittel mit der Kammer (40) in Fluidverbindung stehen, wobei das Gehäuse (12) Mittel zur Beobachtung des Inhaltes seiner Kammer (40) aufweist;

einem in dei Kammer (40) ragenden Mittel (44), das den Durchfluß von Gas und Flüssigkeit in das zweite Zugangsmittel (41) lenkt und

Mitteln (26, 27), die das Verlängerungsmittel (44) wirksam verstopfen, um Flüssigkeit am Einströmen in das Verlängerungsmittel (44) zu hindern, während sie das Einströmen von Gas in das Verlängerungsmittel (44) zulassen, wodurch die zurückgehaltene Flüssigkeit von einem Benutzer durch das Beobachtungsmittel beobachtetwerden kann, wobei das Stöpselmittel einen Entlüftungsstöpsel (26) aufweist, der aus gasdurchlässigem und flüssigkeitsundurchlässigem, vorzugsweise luftdurchlässigem und blutundurchlässigem Material besteht und die Oberfläche des Verlängerungsmittels (44) eng anliegend umschließt und wobei zu dem Stöpselmittel ein flüssigkeitslöslicher Dichtungsstöpsel (27) gehört, der bei Berührung mit der Flüssigkeit von dem Verlängerungsmittel (44) entfernbar ist, damit Flüssigkeit in das Verlängerungsmittel (44) einströmen kann, so daß Flüssigkeit aus dem zweiten Zugangsmittel (41) gesammelt werden kann.

**Revendications**

1. Un dispositif à aiguille pour prise de sang unique (10) permettant de déterminer la pénétration dans ia veine lors d'une prise de sang sur un patient, constitué d':

un logement (12 présentant une extrémité avant (24), une extrémité arrière (25) et une chambre intérieure (40), ledit logement (12) étant translucide au moins autour de la chambre (40) de sorte que ladite chambre (40) peut être vue par l'utilisateur dudit dispositif (10);

un premier trou d'accès (31) traversant l'extrémité avant (24) dudit logement (12) et communiquant avec ladite chambre (40);

une première canule (14) s'étendant vers l'extérieur à partir dudit premier trou d'accès (31) communiquant avec ladite chambre (40) et destinée à être insérée dans le patient;

un second trou d'accès (41) traversant l'extrémité arriére (25) dudit logement et communiquant avec ladite chambre (40);

une seconde canule (15) communiquant avec ledit second trou d'accès (41), pénétrant vers l'intérieur dans ladite chambre (40) et présentant une extrémité fermée et au moins deux trous (45, 48) la traversant;

un tampon d'évent (26) en matériau perméable à l'air et imperméable au sang contact étanche aux liquides avec la surface extérieure de ladite seconde canule (15), ledit tampon d'évent (26) recouvrant l'un desdits trous (48); et

un tampon de scellage soluble dans le sang (27) couvrant ledit second trou (45), de sorte que du sang pénétrant dans ladite chambre (40) à partir de ladite première canule (14) sous pression normale du tourniquet est retardé par ledit tampon de scellage (27) dans sa pénétration dans ladite seconde canule (15) mais peut être vu par l'utilisateur à travers ledit logement translucide (12) pour indiquer la pénétration dans la veine de ladite première canule (14) pendant que l'air éventuellement présent initialement dans ladite chambre (40) traverse ledit tampon d'évent (26) et pénètre dans ledit premier trou (48) pour être évacué dudit dispositif (10),

ledit tampon de scellage (27) étant susceptible de se dissoudre dans le sang présent dans ladite chambre (40) pour démasquer ainsi ledit second trou (45) de façon que le sang puisse traverser ledit second trou (45) et ladite seconde canule (15) pour être recueilli à l'extérieur dudit second trou d'accès (41).

2. Le dispositif de la revendication 1, caractérisé en que ledit premier trou (48) est ménagé à travers le côté de ladite seconde canule (15) et ledit second trou (45) est ménagé à l'extrémite distale de ladite seconde canule (15).

3. Le dispositif de la revendication 1, caractérisé en ce que ledit tampon d'évent (26) forme une poche (50) autour de l'extrémité distale de ladite seconde canule (15) pour faciliter la pose du tampon de scellage (27) sur ledit second trou (45).

4. Le dispositif de la revendication 1, caractérisé en ce que le matériau dudit tampon d'évent (26) est poreux.

5. Le dispositif de la revendication 4, caractérisé en ce que ledit matériau du tampon est du polyéthyléne fritté ayant dans l'ensemble une grandeur de pore nominale d'environ dix (10) micromètres.

6. Le dispositif de la revendication 1, caractérisé en ce que le matériau dudit tampon d'évent (26) est sous forme de cylindre creux.

7. Le dispositif de la revendication 1, charactérisé en ce que ledit tampon de scellage est en polyvinylpyrollidone.

8. Le dispositif de la revendication 1, caractérisé en ce qu'il comporte encore une canule de refoulement s'etendant vers l'extérieur à partir dudit second trou d'accès (41) en communication avec ladite seconde canule (15) et propre à pénétrer un récipient évacué (16) de recueil d'échantillon sanguin.

9. Le dispositif de la revendication 8, caractérisé en ce que ladite canule de refoulement et ladite seconde canule (15) sont réalisées d'un seul tenant en une structure unitaire en une seule pièce.

10. Le dispositif de la revendication 1, caractérisé en ce que tout le logement (12) est translucide.

11. Le dispositif de la revendication 1, caractérisé en ce qu'il comporte encore un support (19) pour un récipient évacué (16) relié audit logement (12).

12. Le dispositif à aiguille pour la détermination de la pénétration du fluide lors du prélévement d'un échantillon de fluide sur une source de fluide caractérisé en ce qu'il comprend: un logement (12) présentant intérieurement une chambre (40) et traversé par un premier (31) et un second (41) moyen d'accès en communication avec ladite chambre (40), ledit logement (12) comportant un moyen permettant de voir le contenu de sa chambre intérieure (40);

un moven (44) se prolongeant à l'intérieur de ladite chambre (40) pour diriger le gaz et le liquide vers l'intérieur dudit second moyen d'accès (41); et

des moyens (26, 27) pour intercepter en pratique ledit moyen de prolongement (44) afin de retarder le passage de liquide dans ce moyen de prolongement (44) tout en laissant du gaz pénétrer dans ce moyen de prolongement (44) de sorte que le liquide retardé peut être vu par l'utilisateur à travers ledit moyen permettant la vision, ledit moyen d'interception comportant un tampon d'évent (26) en matériau perméable aux gaz et imperméable aux liquides et de préférence perméable à l'air et imperméable au sang s'adaptant en contact étroit autour de la surface dudit moyen de prolongement (44) et un tampon de scellage soluble dans les liquides (27) pouvant être éliminé dudit moyen de prolongement (44) lors du contact avec ledit liquide pour permettre au liquide de s'écouler dans ledit moyen de prolongement (44) ce qui permet de recueillir du liquide à partir dudit second moyen d'accès (41).

FIG.1

FIG.2

FIG.4

FIG.3

**FIG.5**